# EUROPÄISCHE PATENTANMELDUNG

(11) **EP 2 090 286 A2**
(43) Veröffentlichungstag der Anmeldung: **19.08.2009**
(21) Anmeldenummer: 08020512.3
(22) Anmeldetag: 26.11.2008
(51) Int. Cl.: A61K 8/35, A61Q 19/06, A61Q 19/00, A61Q 19/08

(54) **Kosmetische oder dermatologische Zubereitungen mit einem Gehalt an 3-(4-Hydroxy-3-methoxyphenyl)-1-(4-hydroxphenyl)-propan-1-on und einem oder mehreren Emulgatoren**

(30) Priorität: 18.02.2008 DE 102008009798
(71) Anmelder: Beiersdorf AG, 20245 Hamburg (DE)
(72) Erfinder: Kolbe, Ludger Dr., 21255 Dohren (DE); Eckert, Julia, 22085 Hamburg (DE); Neufang, Gitta Dr., 20149 Hamburg (DE); Wöhrmann, Micahel Dr., 22525 Hamburg (DE); Knaupmeier, Stefanie, 22391 Hamburg (DE); Raschke, Thomas Dr., 25421 Pinneberg (DE)
(74) Vertreter: Wilke, Jochen

(57) **Zusammenfassung**

Kosmetische oder dermatologische Zubereitungen mit einem Gehalt an 3-(4-Hydroxy-3-methoxyphenyl)-1-(4-hydroxyphenyl)-propan-1-on und einem oder mehreren Emulgatoren.

## Beschreibung

Die vorliegende Erfindung betrifft kosmetische oder dermatologische Zubereitungen mit einem Gehalt an 3-(4-Hydroxy-3-methoxyphenyl)-1-(4-hydroxyphenyl)-propan-1-on und einem oder mehreren Emulgatoren.

Unter Kosmetik kann man alle Maßnahmen zusammenfassen, die aus ästhetischen Gründen Veränderungen an Haut und Haaren vornehmen oder zur Körperreinigung angewendet werden. Kosmetik bedeutet also, das Körperäußere zu pflegen, zu verbessern und zu verschönern, um auf sichtbare, fühlbare und riechbare Weise sowohl den Mitmenschen als auch sich selbst zu gefallen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die chronologische Hautalterung wird z.B. durch endogene, genetisch determinierte Faktoren verursacht. In Epidermis und Dermis kommt es alterungsbedingt z.B. zu folgenden Strukturschäden und Funktionsstörungen, die auch unter den Begriff "Senile Xerosis" fallen können:
a) Trockenheit, Rauhigkeit und Ausbildung von Trockenheitsfältchen,
b) Juckreiz und
c) verminderte Rückfettung durch Talgdrüsen (z.B. nach Waschen).
   Exogene Faktoren, wie UV-Licht und chemische Noxen, können kumulativ wirksam sein und z.B. die endogenen Alterungsprozesse beschleunigen bzw. sie ergänzen. In Epidermis und Dermis kommt es insbesondere durch exogene Faktoren z.B. zu folgenden Strukturschäden- und Funktionsstörungen in der Haut, die über Maß und Qualität der Schäden bei chronologischer Alterung hinausgehen:
d) Sichtbare Gefäßerweiterungen (Teleangiektasien, Cuperosis);
e) Schlaffheit und Ausbildung von Falten;
f) lokale Hyper-, Hypo- und Fehlpigmentierungen (z.B. Altersflecken) und
g) vergrößerte Anfälligkeit gegenüber mechanischem Stress (z.B. Rissigkeit).

Die vorliegende Erfindung betrifft insbesondere Produkte zur Pflege der auf natürliche Weise gealterten Haut, sowie der unter a), e) und g) aufgeführten Phänomene.

Produkte zur Pflege erschlaffter, insbesondere gealterter Haut sind an sich bekannt. Sie enthalten z.B. Retinoide (Vitamin A-Säure und/oder deren Derivate) bzw. Vitamin A und/oder dessen Derivate. Ihre Wirkung auf die Strukturschäden ist allerdings umfangsmäßig begrenzt. Daüber hinaus gibt es bei der Produktentwicklung erhebliche Schwierigkeiten, die Wirkstoffe in ausreichendem Maße gegen oxidativen Zerfall zu stabilisieren. Die Verwendung Vitamin A-Säure-haltiger Produkte bedingt darüber hinaus oft starke erythematöse Hautreizungen. Retinoide sind daher nur in geringen Konzentrationen einsetzbar.

Oftmals wird mit der erschlafften Haut auch eine Begleiterscheinung der Übergewichtigkeit und/oder der damit häufig einhergehenden sogenannten Cellullite verbunden.

Das Körperbewußtsein der Verbraucher ist in den vergangenen Jahren deutlich gestiegen. Dabei werden neben reinigenden und pflegenden Anwendungen auch zunehmend Maßnahmen ergriffen, um die Körpersilhouette zu verbessern. Die Cellulite - ein weit verbreitetes Phänomen - nimmt dabei eine zentrale Stellung ein. Das sichtbare Bild der Cellulite beruht auf einer Zunahme von Fettpolstern in der Subcutis (Unterhautfettgewebe), einer Bindegewebsschwäche sowie einer Minderung der Durchströmungsverhältnisse in den Blut- und Lymphbahnen. Die Ursache ist somit eine zum Teil anlagebedingte Schwächung des Bindegewebes mit gleichzeitigem Auftreten von vergrößerten Fettzellkammern infolge von Übergewicht, unausgewogener Ernährung, Bewegungsmangel. Die Entstehung der Cellulite kann ferner auf eine erhöhte Durchlässigkeit der Haargefäßwände zurückgeführt werden, die das Eindringen von Wasser ins Bindegewebe erlaubt.

Daneben kann an den betroffenen Hautbereichen ein lokaler Mangel an Testosteron bestehen. Jedenfalls ist Cellulite eine Erscheinung die fast niemals bei Männern zu beobachten ist.

Aufgabe der vorliegenden Erfindung war es somit, Wege zu finden, die die Nachteile des Standes der Technik vermeiden. Insbesondere war es eine Aufgabe der vorliegenden Erfindung, Zubereitungen zur Verfügung zu stellen, die eine vorteilhafte Straffung erschlaffter Haut bewirken können.

Eine weitere Aufgabe der vorliegenden Erfindung war es, hautpflegende Zubereitungen und Zubereitungen zur Pflege der Haut zur Verfügung zu stellen, wobei die Zubereitungen das Körperäußere pflegen und verschönern sollten. Dabei sollten die Zubereitungen insbesondere auch die Hautfeuchtigkeit verbessern.

Es hat sich überraschenderweise herausgestellt, daß eine kosmetische Zubereitung, dadurch gekennzeichnet, daß sie eine kosmetisch wirksame Menge an 3-(4-Hydroxy-3-methoxyphenyl)-1-(4-hydroxyphenyl)-propan-1-on und ein oder mehreren Emulgatoren enthält, den Nachteilen des Standes der Technik abhilft.

Erfindungsgemäß wird eine vom pH-Wert unabhängige Löslichkeit von 3-(4-Hydroxy-3-methoxyphenyl)-1-(4-hydroxyphenyl)-propan-1-on erreicht.

Erfindungsgemäße, als Emulsionen vorliegende Zubereitungen enthalten einen oder mehrere Emulgatoren. O/W-EMulgatoren können beispielsweise vorteilhaft gewählt werden aus der Gruppe der polyethoxylierten bzw. polypropoxylierten bzw. polyethoxylierten und polypropoxylierten Produkte, z.B.:
- der Fettalkoholethoxylate
- der ethoxylierten Wollwachsalkohole,
- der Polyethylenglycolether der allgemeinen Formel R-O-(-CH₂-CH₂-O-)ₙ-R',
- der Fettsäureethoxylate der allgemeinen Formel
   R-COO-(-CH₂-CH₂-O-)ₙ -H,
- der veretherten Fettsäureethoxylate der allgemeinen Formel
   R-COO-(-CH₂-CH₂-O-)ₙ -R',
- der veresterten Fettsäureethoxylate der allgemeinen Formel
   R-COO-(-CH₂-CH₂-O-)ₙ -C(O)-R',
- der Polyethylenglycolglycerinfettsäureester
- der ethoxylierten Sorbitanester
- der Cholesterinethoxylate
- der ethoxylierten Triglyceride
- der Alkylethercarbonsäuren der allgemeinen Formel
   R-O-(-CH₂-CH₂-O-)ₙ-CH₂-COOH nd n eine Zahl von 5 bis 30 darstellen,
- der Polyoxyethylensorbitolfettsäureester,
- der Alkylethersulfate der allgemeinen Formel R-O-(-CH₂-CH₂-O-)ₙ-SO₃-H
- der Fettalkoholpropoxylate der allgemeinen Formel
   R-O-(-CH₂-CH(CH₃)-O-)ₙ-H,
- der Polypropylenglycolether der allgemeinen Formel
   R-O-(-CH₂-CH(CH₃)-O-)ₙ-R',
- der propoxylierten Wollwachsalkohole,
- der veretherten Fettsäurepropoxylate
   R-COO-(-CH₂-CH(CH₃)-O-)ₙ-R',
- der veresterten Fettsäurepropoxylate der allgemeinen Formel
   R-COO-(-CH₂-CH(CH₃)-O-)ₙ-C(O)-R',
- der Fettsäurepropoxylate der allgemeinen Formel
   R-COO-(-CH₂-CH(CH₃)-O-)ₙ-H,
- der Polypropylenglycolglycerinfettsäureester
- der propoxylierten Sorbitanester
- der Cholesterinpropoxylate
- der propoxylierten Triglyceride
- der Alkylethercarbonsäuren der allgemeinen Formel
   R-O-(-CH₂-CH(CH₃)O-)ₙ-CH₂-COOH
- der Alkylethersulfate bzw. die diesen Sulfaten zugrundeliegenden Säuren der allgemeinen Formel R-O-(-CH₂-CH(CH₃)-O-)ₙ-SO₃-H
- der Fettalkoholethoxylate/propoxylate der allgemeinen Formel
   R-O-Xₙ-Yₘ-H,
- der Polypropylenglycolether der allgemeinen Formel
   R-O-Xₙ-Yₘ-R',
- der veretherten Fettsäurepropoxylate der allgemeinen Formel
   R-COO-Xₙ-Yₘ-R',
- der Fettsäureethoxylate/propoxylate der allgemeinen Formel
   R-COO-Xₙ-Yₘ-H,

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11 - 18, ganz besonders vorteilhaft mit mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:

Polyethylenglycol(2)stearylether (Steareth-2), Polyethylenglycol(14)stearylether (Steareth-14), Polyethylenglycol(16)stearylether (Steareth-16), Polyethylenglycol(17)stearylether (Steareth-17), Polyethylenglycol(18)stearylether (Steareth-18), Polyethylenglycol(19)stearylether (Steareth-19), Polyethylenglycol(20)stearylether (Steareth-20), Polyethylenglycol(21)stearylether (Steareth-21),

Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol-(15)isostearylether (Isosteareth-15), Polyethylenglycol(16)isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol-(20)isostearylether (Isosteareth-20),

Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20),

Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)-isocetylether (Isoceteth-16), Polyethylenglycol(17)isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20),

Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15),

Polyethylenglycol(12)laurylether (Laureth-12), Polyethylenglycol(12)isolaurylether (Isolaureth-12).

Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)-cetylstearylether (Ceteareth-16), Polyethylenglycol(17)cetylstearylether (Ceteareth-17), Polyethylenglycol(18)cetylstearylether (Ceteareth-18), Polyethylenglycol(19)cetylstearylether (Ceteareth-19), Polyethylenglycol(20)cetylstearylether (Ceteareth-20),

Es ist ferner von Vorteil, die Fettsäureethoxylate aus folgender Gruppe zu wählen:

Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat,

Polyethylenglycol(12)isostearat, Polyethylenglycol(13)isostearat, Polyethylenglycol(14)isostearat, Polyethylenglycol(15)isostearat, Polyethylenglycol(16)isostearat, Polyethylenglycol-(17)isostearat, Polyethylenglycol(18)isostearat, Polyethylenglycol(19)isostearat, Polyethylenglycol(20)isostearat, Polyethylenglycol(21)isostearat, Polyethylenglycol(22)isostearat, Polyethylenglycol(23)isostearat, Polyethylenglycol(24)isostearat, Polyethylenglycol(25)isostearat, Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol-(18)oleat, Polyethylenglycol(19)oleat, Polyethylenglycol(20)oleat

Als ethoxylierte Alkylethercarbonsäure bzw. deren Salz kann vorteilhaft das Natriumlaureth-11-carboxylat verwendet werden.

Als Alkylethersulfat kann Natrium Laureth 1-4 sulfat vorteilhaft verwendet werden.

Als ethoxyliertes Cholesterinderivat kann vorteilhaft Polyethylenglycol(30)Cholesterylether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/caprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 - 18 C-Atomen, Diglycerinester gesättigter und/oder ungesättigter, verzweigter und/oder unverzweigter Alkancarbonsäuren einer Kettenlänge von 8 bis 24, insbesondere 12 - 18 C-Atomen, Monoglycerinether gesättigter und/oder ungesättigter, verzweigter und/oder unverzweigter Alkohole einer Kettenlänge von 8 bis 24, insbesondere 12 - 18 C-Atomen, Diglycerinether gesättigter und/oder ungesättigter, verzweigter und/oder unverzweigter Alkohole einer Kettenlänge von 8 bis 24, insbesondere 12 - 18 C-Atomen, Propylenglycolester gesättigter und/oder ungesättigter, verzweigter und/oder unverzweigter Alkancarbonsäuren einer Kettenlänge von 8 bis 24, insbesondere 12 - 18 C-Atomen sowie Sorbitanester gesättigter und/oder ungesättigter, verzweigter und/oder unverzweigter Alkancarbonsäuren einer Kettenlänge von 8 bis 24, insbesondere 12 - 18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.
Besonders vorteilhaft kann der Emulgator oder können die Emulgatoren gewählt werden aus der Gruppe der grenzflächenaktiven Substanzen A, gewählt aus der Gruppe der Glucosederivate, welche sich durch die Strukturformel 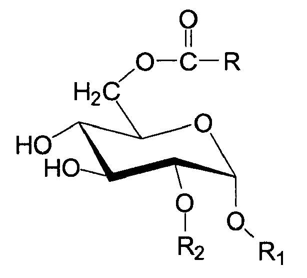 auszeichnen, wobei R einen verzweigten oder unverzweigten Alkylrest mit 1 bis 24 Kohlenstoffatomen darstellt, wobei R₁ entweder ein Wasserstoffatom oder einen verzweigten oder unverzweigten Alkylrest mit 1 bis 24 Kohlenstoffatomen darstellt und wobei R₂ entweder ein Wasserstoffatom oder einen verzweigten oder unverzweigten Acylrest mit 1 bis 24 Kohlenstoffatomen darstellt, enthalten,

Ebenfalls äußerst vorteilhaft kann der Emulgator oder können die Emulgatoren gewählt werden aus der Gruppe der grenzflächenaktiven Substanzen B, gewählt aus der Gruppe der Substanzen der allgemeinen Strukturformel 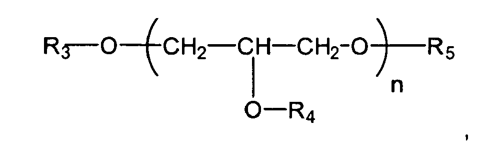 wobei R₃, R₄ und R₅ voneinander gewählt werden aus der Gruppe, welche umfaßt: H, verzweigte bzw. unverzweigte, gesättigte bzw. ungesättigte Fettsäurereste mit 8 bis 24 Kohlenstoffatomen, bei welchen bis zu drei aliphatische Wasserstoffatome durch Hydroxygruppen substituiert sein können und n eine Zahl von 2 bis 8 darstellt.

Besonders vorteilhaft wird oder werden die grenzflächenaktiven Substanzen A aus der Gruppe Methylglucosemonostearat (Formel wie nachstehend)

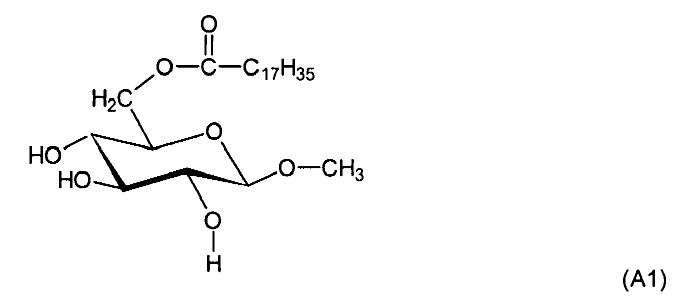

Methylglucosedistearat (Formel wie nachstehend) 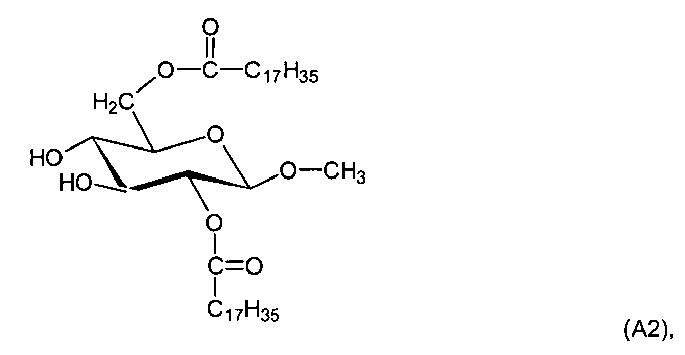 und beliebigen Gemischen daraus, beispielsweise ungefähr äquimolaren Mischungen daraus gewählt, welche auch als Methylglucosesesquistearat bezeichnet werden. Solches Methylglucosesesquistearat ist im Handel beispielsweise unter der Warenbezeichnung Tego® Care PS von der Geselischaft.Goldschmidt AG erhältlich.

Besonders vorteilhaft wird oder werden die grenzflächenaktiven Substanzen B gewählt aus der Gruppe der Verbindungen, bei welcher n den Wert 3 annimmt und R₃, R₄ und R₅ unabhängig voneinander gewählt werden aus der Gruppe, welche umfaßt: H, verzweigte bzw. unverzweigte, gesättigte bzw. ungesättigte Fettsäurereste mit 14 bis 20 Kohlenstoffatomen, insbesondere die nachfolgend aufgeführten Strukturen:

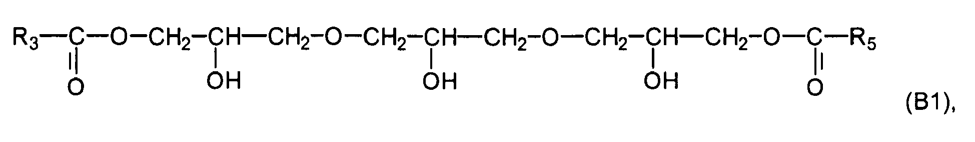

Als erfindungsgemäß bevorzugte Emulgatorkombination hat sich ein ungefähr äquimolekulares Gemisch aus den Verbindungen A2 und B1, wobei in B1 die Reste R₃ und R₅ vorzugsweise beide einen Stearatrest bezeichnen, herausgestellt. Solche Emulgatorkombinationen sind als "Polyglyceryl(3)-Methylglucosedistearat" (PGMS) unter der Warenbezeichnung Tego Care® 450 von der Gesellschaft Goldschmidt AG erhältlich.

Vorteilhaft sind ferner die Emulgatoren Cetearylglucosid (zum Beispiel unter der Warenbezeichnung Tego Care® CG 90 von der Gesellschaft Evonik Goldschmidt AG oder unter der Warenbezeichnung Emulgade® PL 68/50 von der Gesellschaft Cognis erhältlich), Methylglucosesesquistearat, Natrium Cetearylsulfat, Stearinsäure, Triceteareth-4-Phosphate Natrium Stearoylglutamat (zum Beispiel unter der Warenbezeichnung Emulgin® SG von der Gesellschaft Cognis erhältlich), Stearinsäure, Palmitinsäure sowie Glycerylstearatcitrat (zum Beispiel unter der Warenbezeichnung Imwitor® 372 von der Gesellschaft Sasol erhältlich).

Vorteilhaft ist ferner der polymere Emulgator Acrylates /C₁₀₋₃₀ Alkyl/Acrylate Crosspolymer (unter der Warenbezeichnung Pemulen® TR1 von der Gesellschaft Noveon erhältlich)

Die Gesamtmenge an einem oder mehreren Emulgatoren wird in den fertigen kosmetischen oder dermatologischen Zubereitungen vorteilhaft 0,001 bis 10 Gew.-%, insbesondere 0,05 bis 5 Gew.-%, ganz besonders 0,01 bis 3 Gew.%, bezogen auf das Gesamtgewicht der Zubereitungen, gewählt.

Das Verhältnis von 3-(4-Hydroxy-3-methoxyphenyl)-1-(4-hydroxyphenyl)-propan-1-on zu Polyol (bzw. der Gesamtmenge an Emulgatoren) sollte 1:10 bis 1:1000 betragen.

Die Verwendung erfindungsgemäßer Zubereitungen mit einem kosmetisch wirksamen Gehalt an 3-(4-Hydroxy-3-methoxyphenyl)-1-(4-hydroxyphenyl)-propan-1-on - z. B. in Form der genannten Beispiele - führt in überraschender Weise zu einer erheblichen Verbesserung des Aussehens der Haut, insbesondere werden durch Anwenden erfindungsgemäßer Zubereitungen
- die Barriereeigenschaften der Haut erhalten oder wiederhergestellt,
- besser der Hautaustrocknung entgegengewirkt sowie
- die Haut besser vor Umwelteinflüssen geschützt.

Durch die Verwendung erfindungsgemäßer Zubereitungen wird ferner die Hautfeuchtigkeit und die Tiefenfeuchtigkeit der Haut gesteigert, wobei diese hautbefeuchtende Pflege-Wirkung und der Schutz vor Feuchtigkeitsverlust über mehrere Stunden bzw. sogar über den ganzen Tag oder noch länger anhält.

Erfindungsgemäß ist daher auch die Verwendung kosmetischer Zubereitungen, die eine kosmetisch wirksame Menge an 3-(4-Hydroxy-3-methoxyphenyl)-1-(4-hydroxyphenyl)-propan-1-on enthalten, zur Steigerung der Hautfeuchtigkeit bzw. zur Befeuchtung der Haut.

Die erfindungsgemäßen Zubereitungen eignen sich darüber hinaus hervorragend zur Pflege sensibler Haut.

Es war ferner überraschend, dass durch Anwenden erfindungsgemäßer Zubereitungen die Elastizität der Haut, der Haare und/oder der Nägel und daher Cellulite bzw. das Erscheinungsbild der sog. "Orangenhaut" verbessert wird.

Bevorzugt enthalten kosmetische oder dermatologische Zubereitungen gemäß der Erfindung 0,001 bis 30 Gew.-%, bevorzugt 0,1 bis 15 Gew.-%, besonders bevorzugt 0,5 bis 5 Gew.-% an 3-(4-Hydroxy-3-methoxyphenyl)-1-(4-hydroxyphenyl)-propan-1-on, bezogen auf das Gesamtgewicht der Zubereitung.

Herstellungsverfahren für das 3-(4-Hydroxy-3-methoxyphenyl)-1-(4-hydroxyphenyl)-propan-1-on 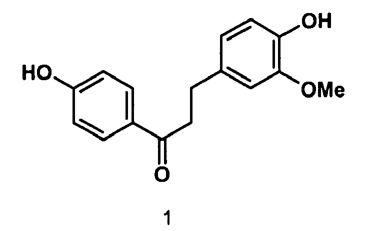 können vorteilhaft aus den folgenden Schritten bestehen oder diese umfassen:
a) Umsetzung von p-Hydroxyacetophenon mit Vanillin zu 3-(4-Hydroxy-3-methoxyphenyl)-1-(4-hydroxyphenyl)-2-propen-1-on und
b) Hydrierung des in Schritt a) gewonnenen 3-(4-Hydroxy-3-methoxyphenyl)-1-(4-hydroxyphenyl)-2-propen-1-on zu 3-(4-Hydroxy-3-methoxyphenyl)-1-(4-hydroxyphenyl)-propan-1-on.

In einer bevorzugten Ausgestaltung des erfindungsgemäßen Herstellverfahrens für die Verbindung der Formel 1 wird die Umsetzung von p-Hydroxyacetophenon mit Vanillin in Schritt a) so durchgeführt, daß p-Hydroxyacetophenon und Vanillin einer Mischung aus Kaliumhydroxid in Diethylenglykoldiethylether bei erhöhter Temperatur zugesetzt wird. Die Temperatur liegt üblicherweise im Bereich von 50 bis 200 °C, bevorzugt im Bereich von 80°C bis 140°C, weiter bevorzugt im Bereich von 100 bis 120°C.

Anschließend wird die entstandene Verbindung bevorzugt zu 3-(4-Hydroxy-3-methoxyphenyl)-1-(4-hydroxyphenyl)-2-propen-1-on hydrolysiert. Bevorzugt wird der pH-Wert im Bereich von 6 bis 7 eingestellt.

In einer bevorzugten Ausgestaltung des erfindungsgemäßen Herstellverfahrens für die Verbindung der Formel 1 wird die Hydrierung des in Schritt (a) gewonnenen 3-(4-Hydroxy-3-methoxyphenyl)-1-(4-hydroxyphenyl)-2-propen-1-on zu 3-(4-Hydroxy-3-methoxyphenyl)-1-(4-hydroxyphenyl)-propan-1-on so durchgeführt, daß das entstandene 3-(4-Hydroxy-3-methoxyphenyl)-1-(4-hydroxyphenyl)-2-propen-1-on in Tetrahydrofuran gelöst und mit einer geeigneten Menge Pd auf Aktivkohle (Pd-Gehalt: 5 Gew.-%, Wassergehalt ca. 50 Gew.-%, jeweils bezogen auf die Gesamtmasse des Katalysators) versetzt wird. Vorzugsweise wird die Reaktion bei Normaldruck und weiter vorzugsweise bei Raumtemperatur (ca. 20°C) vorgenommen.

### Synthesebeispiel für 3-(4-Hydroxy-3-methoxyphenyl)-1-(4-hydroxyphenyl)-1-propanon

### (a) Umsetzung von p-Hydroxyacetophenon mit Vanillin:

Es werden 20 g Kaliumhydroxid in 100 g Diethylenglykoldiethylether vorgelegt, auf 120°C unter Rühren erhitzt und mit einer Mischung von 14 g p-Hydroxyacetophenon und 15 g Vanillin innerhalb von 1 h versetzt. Nach beendeter Dosierung rührt man noch 20 min nach, hydrolysiert und stellt auf pH 6-7 ein. Nach Phasentrennung entfernt man das Lösungsmittel und erhält 25 g 3-(4-Hydroxy-3-methoxyphenyl)-1-(4-hydroxyphenyl)-2-propen-1-on, Ausbeute: 93% der Theorie.

### (b) Hydrierung des in Schritt (a) gewonnenen Reaktionsprodukts:

10 g 3-(4-Hydroxy-3-methoxyphenyl)-1-(4-hydroxyphenyl)-2-propen-1-on werden in 100 g Tetrahydrofuran gelöst, mit 0,2 g Pd auf Aktivkohle (Pd-Gehalt: 5 Gew.-%, Wassergehalt ca. 50 Gew.-%, jeweils bezogen auf die Gesamtmasse des Katalysators) versetzt und bei Normaldruck und Raumtemperatur (ca. 20°C) hydriert. Man erhält nach Entfernung des Katalysators und des Lösungsmittels 9 g an 3-(4-Hydroxy-3-methoxyphenyl)-1-(4-hydroxyphenyl)-propan-1-on. Ausbeute: 89% der Theorie

Spektroskopische Daten von 3-(4-Hydroxy-3-methoxyphenyl)-1-(4-hydroxyphenyl)-propan-1-on (Formel 1):
¹³C-NMR (CDCl₃; 75,5 MHz): δ (ppm) = 197,42(s), 161,85(s), 147,24(s), 144,44(s), 132,03(s), 130,37(d), 130,37(d), 128,18(s), 120,27(d), 115,11(d), 115,07(d), 115,07(d), 112,53(d), 55,42(q), 39,30(t), 29,42(t);
MS: m/z (%) = M⁺-Ion 272(82), 151(24), 137(77), 121(100), 93(19), 65(22).

Die kosmetischen oder dermatologischen Zubereitungen können erfindungsgemäß wie üblich zusammengesetzt sein und zur Behandlung, der Pflege und der Reinigung der Haut und/oder der Haare und als Schminkprodukt in der dekorativen Kosmetik dienen.

Sie enthalten bevorzugt 0,001 Gew.-% bis 10 Gew.-%, bevorzugt 0,05 Gew.-% bis 5 Gew.-%, insbesondere 0,1 - 2,0 Gew.-%, bezogen auf das Gesamtgewicht der Zubereitungen, an 3-(4-Hydroxy-3-methoxyphenyl)-1-(4-hydroxyphenyl)-propan-1-on.

Die kosmetischen oder dermatologischen Zubereitungen enthalten ferner bevorzugt 0,001 Gew.-% bis 30 Gew.-%, bevorzugt 0,05 Gew.-% bis 10 Gew.-%, insbesondere 0,1 - 2,0 Gew.-%, bezogen auf das Gesamtgewicht der Zubereitungen, an einem oder mehreren Emulgatoren.

Erfindungsgemäße kosmetische und dermatologische Zubereitungen können in verschiedenen Formen vorliegen. So können sie z.B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsionen, beispielsweise vom Typ Wasser-in-Öl-in-Wasser (W/O/W), ein Gel, einen festen Stift, eine Salbe oder auch ein Aerosol darstellen. Es ist auch erfindugsgemäß vorteilhaft, 3-(4-Hydroxy-3-methoxyphenyl)-1-(4-hydroxyphenyl)-propan-1-on in verkapselter Form darzureichen, z.B. in Kollagenmatrices und anderen üblichen Verkapselungsmaterialien, z.B. als Cellulose- oder Wachsverkapselungen, in Gelatine, Wachsmatrices oder liposomal verkapselt. Insbesondere Wachsmatrices wie sie in der DE-OS 43 08 282 beschrieben werden, haben sich als günstig herausgestellt.

Es ist auch möglich und vorteilhaft im Sinne der vorliegenden Erfindung, 3-(4-Hydroxy-3-methoxyphenyl)-1-(4-hydroxyphenyl)-propan-1-on und/oder deren Derivate in wäßrige Systeme bzw. Tensidzubereitungen zur Reinigung der Haut und der Haare einzufügen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polymere, Schaumstabilisatoren, Elektrolyte, organische Lösemittel oder Siliconderivate.

Vorteilhaft werden die Antioxidantien gewählt aus der Gruppe bestehend α-Hydroxysäuren (z.B. Citronensäure, Milchsäure, Apfelsäure), EDTA, Ubichinon und Ubichinol und deren Derivate (insbesondere Ubichinon Q10), Vitamin C und Derivate (z.B. Ascorbylpalmitat, Na-Ascorbylphosphat), Tocopherole und Derivate (z.B. Vitamin-E-acetat), Rutinsäure und deren Derivate insbesondere α-Glycosylrutin, Genistein, Butylhydroxytoluol.

Die Menge an Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Siliconöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Die wäßrige Phase der erfindungsgemäßen Zubereitungen enthält gegebenenfalls vorteilhaft Alkohole, Diole oder Emulgatoren niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Carageenan, Hydroxypropylmethylcellulose, Ammonium Polyacryloyldimethyltaurat, Ammonium Polyacryldimethyltaurid/Vinylformamid-copolymer, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z.B. die genannten Fette, Öle, Wachse und anderen Fettkörper, sowie Wasser und einen oder mehrere Emulgatoren.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Als Treibmittel für erfindungsgemäße, aus Aerosolbehältern versprühbare Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, Gewichtsprozente, auf das Gewicht und die Gesamtmenge bzw. auf das Gesamtgewicht der Zubereitungen bezogen.

| **Beispiel 1** | **Gew.-%** |
|---|---|
| O/W-Nachtcreme | |
| Glycerylstearatcitrat | 2 |
| Stearylalkohol | 2 |
| Cetylalkohol | 2 |
| Hydrierte Kokosfettglyceride (Hydrogenated Coco Glycerides) | 2 |
| Caprylsäure/Caprinsäuretriglyceride | 2 |
| Octyldodecanol | 2 |
| Cyclometicone | 3 |
| Dicaprylylether | 2 |
| Tocopherylacetat | 0,5 |
| Ubichinon (Q10) | 0,1 |
| Natriumascorbylphosphat | 0,1 |
| 3-(4-Hydroxy-3-methoxyphenyl)-1-(4-hydroxyphenyl)-propan-1-on | 0,2 |
| Urea | 0,5 |
| Ceramid III | 0,1 |
| Taurin | 0,5 |
| Cholesterin | 0,3 |
| p-Hydroxybenzoesäurealkylester (Paraben) | 0,4 |
| Ethylhexylglycerin | 0,5 |
| Carrageenan | 0,3 |
| Carbomer | 0,2 |
| EDTA | 0,2 |
| Glycerin | 8 |
| wasser- und/oder öllösliche Farbstoffe | 0,1 |
| Füllstoffe/ Additive (Nylon, BHT) | 1 |
| Parfüm | q.s. |
| Wasser | ad 100 |

| **Beispiel 2** | **Gew.-%** |
|---|---|
| O/W-Tagescreme | |
| Steareth-21 | 3 |
| Steareth-2 | 1 |
| Stearylalkohol | 1 |
| Myristylmyristate | 1 |
| C₁₂₋₁₅ Alkylbenzoat | 3 |
| Cyclometicone | 5 |
| Mineralöl | 5 |
| Jojobaöl | 3 |
| Dicarprylylcarbonat | 3 |
| Ethylhexylcyanodiphenylacrylat (Octocrylen) | 3 |
| Bis-Ethylhexyloxyphenol-methoxyphenyltriazin | 1 |
| Citronensäure, Natriumsalz | 0.1 |
| 3-(4-Hydroxy-3-methoxyphenyl)-1-(4-hydroxyphenyl)-propan-1-on | 0,05 |
| Folsäure | 0,1 |
| Phenoxyethanol | 0,5 |
| Hexamidindiisethionat | 0,04 |
| 1,3-Dimethylol-5,5-dimethyl-hydantoin(DMDM Hydantoin) | 0,1 |
| EDTA | 0,2 |
| Ethanol (vergällt) | 2 |
| Carbomer | 0,3 |
| Glycerin | 10 |
| Additive (Distärkephosphat, BHT) | 1 |
| Parfüm | q.s. |
| Wasser | ad 100 |

| **Beispiel 3** | **Gew.-%** |
|---|---|
| Sonnenschutzcreme | |
| Glycerylstearat | 3 |
| PEG-40-Stearat | 1 |
| Cetylalkohol | 3 |
| C₁₂₋₁₅ Alkylbenzoat | 3 |
| Hydrierte Kokosfettglyceride (Hydrogenated Coco Glycerides) | 1 |
| Octyldodecanol | 3 |
| Cyclometicone | 3 |
| Ethylhexylmethoxycinnamat | 5 |
| Phenylbenzimidazol sulfonsäure | 1 |
| Butylmethoxydibenzoylmethan | 2 |
| Niacinamid | 0,1 |
| Tocopherylacetat | 1 |
| 3-(4-Hydroxy-3-methoxyphenyl)-1-(4-hydroxyphenyl)-propan-1-on | 0,5 |
| Methylpropandiol | 1 |
| Phenoxyethanol | 0,5 |
| p-Hydroxybenzoesäurealkylester (Paraben) | 0,2 |
| Diazolidinylharnstoff | 0,1 |
| Carbomer (Ultrez 10) | 0,1 |
| Xanthan Gum | 0,2 |
| Glycerin | 8 |
| EDTA | 0,2 |
| Additive (BHT) | 0,1 |
| Parfüm | q.s. |
| Wasser | ad 100 |

| **Beispiel 4** | **Gew.-%** |
|---|---|
| O/W-Creme | |
| Glycerylstearat | 1 |
| PEG-100-Stearat | 3 |
| Stearylalkohol | 3 |
| C₁₂₋₁₅ Alkylbenzoat | 2 |
| Caprylsäure/Caprinsäuretriglyceride | 2 |
| Macadamiaöl | 1 |
| Myristylmyristat | 2 |
| Dimethicon | 2 |
| Bis-Ethylhexyloxyphenolmethoxyphenyltriazin | 1 |
| Ethylhexylcyanodiphenylacrylat (Octocrylen) | 5 |
| Creatin | 0,5 |
| 3-(4-Hydroxy-3-methoxyphenyl)-1-(4-hydroxyphenyl)-propan-1-on | 0,1 |
| Panthenol | 1 |
| Phenoxyethanol | 0,4 |
| p-Hydroxybenzoesäurealkylester (Paraben) | 0,3 |
| Ginseng Extrakt | 0,2 |
| Carragenan | 0,3 |
| Carbomer | 0,1 |
| Glycerin | 5 |
| Butylenglycol | 3 |
| Methylpropandiol | 1 |
| Additive (SiO₂, Talkum) | 0,5 |
| Parfüm | q.s. |
| Wasser | ad 100 |

| **Beispiel 5** | **Gew.-%** |
|---|---|
| Sun Spray | |
| Ceteareth-20 | 1 |
| Butylenglycol Dicaprylat/Dicaprat | 3 |
| Caprylsäure/Caprinsäuretriglyceride | 2 |
| Octyldodecanol | 1 |
| Dicaprylylcarbonat | 2 |
| Dimethicon | 2 |
| Butylmethoxydibenzoylmethan | 2 |
| Ethylhexylmethoxycinnamat | 5 |
| Phenylbenzimidazol sulfonsäure | 1 |
| 3-(4-Hydroxy-3-methoxyphenyl)-1-(4-hydroxyphenyl)-propan-1-on | 0,5 |
| Tocopherylacetat | 0,05 |
| EDTA | 0,2 |
| Ethanol | 2 |
| Phenoxyethanol | 0,3 |
| p-Hydroxybenzoesäurealkylester (Paraben) | 0,4 |
| Acrylates /C₁₀₋₃₀ Alkyl/Acrylate Crosspolymer | 0,2 |
| VP/Hexadecene Copolymer | 0,3 |
| Glycerin | 5 |
| Parfüm | q.s. |
| Wasser | ad 100 |

| **Beispiel 6** | **Gew.-%** |
|---|---|
| O/W Lotion | |
| Polyglyceryl-3-Methylglucosedistearat | 3 |
| Cetylalkohol | 1 |
| C₁₂₋₁₅ Alkylbenzoat | 3 |
| Butylenglycol Dicaprylat/Dicaprat | 2 |
| Hydriertes Polydecen | 1 |
| Dimethylpolysiloxan (Dimethicon) | 1 |
| Isodecylneopentanoat | 3 |
| 3-(4-Hydroxy-3-methoxyphenyl)-1-(4-hydroxyphenyl)-propan-1-on | 0,2 |
| Arginin | 0,2 |
| Phytosterole | 0,1 |
| EDTA | 0,2 |
| Phenoxyethanol | 0,4 |
| lodopropinylbutylcarbamat | 0,05 |
| p-Hydroxybenzoesäurealkylester (Paraben) | 0,4 |
| Carbomer 980 | 0,2 |
| Glycerin | 5 |
| Additive (Distärkephosphat, Talkum, BHT) | 2 |
| Parfüm | q.s. |
| Wasser | ad 100 |

| **Beispiel 7** | **Gew.-%** |
|---|---|
| Hydrodispersion | |
| Carbomer 981 | 0.2 |
| Acrylates /C₁₀₋₃₀ Alkyl/Acrylate Crosspolymer | 0.3 |
| Xanthan Gum | 0,1 |
| C₁₂₋₁₅ Alkylbenzoat | 2 |
| Cyclometicon | 5 |
| Dimethylpolysiloxan (Dimethicon) | 1 |
| Coffein | 0,3 |
| 3-(4-Hydroxy-3-methoxyphenyl)-1-(4-hydroxyphenyl)-propan-1-on | 0,1 |
| Carnitin | 0,2 |
| EDTA | 0,2 |
| Phenoxyethanol | 0,4 |
| Fucus vesiculosus | 0,1 |
| p-Hydroxybenzoesäurealkylester (Paraben) | 0,3 |
| Butylenglycol | 3 |
| Glycerin | 5 |
| Additive (Farbstoff) | 2 |
| Parfüm | q.s. |
| Wasser | ad 100 |

| **Beispiel 8** | **Gew.-%** |
|---|---|
| Whitening-Lotion | |
| Glycerylstearat SE | 2 |
| Natrium Cetearyl Sulfat | 0,5 |
| Cetearylalkohol | 3 |
| C₁₂₋₁₅ Alkylbenzoat | 2 |
| Caprylsäure/Caprinsäuretriglyceride | 2 |
| Cyclometicone | 3 |
| Dimethicon | 1 |
| Butylmethoxydibenzoylmethan | 0,5 |
| Ethylhexylmethoxycinnamat | 3 |
| Ellagsäure | 0,1 |
| Tocopherylacetat | 0,5 |
| 3-(4-Hydroxy-3-methoxyphenyl)-1-(4-hydroxyphenyl)-propan-1-on | 0,1 |
| Dioic Acid | 0,5 |
| Phenoxyethanol | 0,4 |
| p-Hydroxybenzoesäurealkylester (Paraben) | 0,3 |
| Methylpropandiol | 2 |
| Gingko Extrakt | 0,2 |
| Carragenan | 0,2 |
| Carbomer | 0,1 |
| Glycerin | 5 |
| Methylpropandiol | 1 |
| Additive (BHT, Natrium Stärke octenyl succinat) | 0,5 |
| Parfüm | q.s. |
| Wasser | ad 100 |

| **Beispiel 9** | |
|---|---|
| **Deodorant / Antitranspirant- Roll-on** | |
| 3-(4-Hydroxy-3-methoxyphenyl)-1-(4-hydroxyphenyl)-propan-1-on | 0,1 |
| Polyethylenglykol(21)stearylether | 3 |
| Polyethylenglykol(2)stearylether | 2 |
| Polypropylenglykol(15)stearylether | 2 |
| EDTA | 0,10 |
| Avocadoöl | 0,10 |
| Parfum, Antioxidantien | q.s. |
| 2-Ethylhexylglycerinether (Octoxyglycerin) | 0,50 |
| Hexandiol | 3 |
| Aluminiumchlorohydrat | 10 |
| Wasser | ad 100 |

## Patentansprüche

1. Kosmetische oder dermatologische Zubereitungen mit einem Gehalt an 3-(4-Hydroxy-3-methoxyphenyl)-1-(4-hydroxyphenyl)-propan-1-on und einem oder mehreren Emulgatoren.

2. Zubereitungen nach Anspruch 1, **dadurch gekennzeichnet, daß** der oder die Emulgatoren gewählt werden aus der Gruppe der anionischen und nichtionischen O/W-Emulgatoren.

3. Zubereitungen nach Anspruch 1, **dadurch gekennzeichnet, daß** der oder die Emulgatoren gewählt werden aus der Gruppe der polymeren Emulgatoren

4. Zubereitungen nach Anspruch 2, **dadurch gekennzeichnet, daß** der oder die anionischen und nichtionischen Emulgatoren gewählt werden aus der Gruppe Glycerylstearatcitrat, Polyglyceryl-3-methylglucosedistearat, PEG-40-Steartat, PEG-100-Stearat, Ceteareth-20, Steareth-2, Steareth-21, Cetearylglucoside, Natrium Cetearylsulfat, Stearinsäure, Natrium Stearoylglutamat

5. Zubereitungen nach Anspruch 3, **dadurch gekennzeichnet, daß** der oder die polymeren Emulgatoren gewählt werden aus der Gruppe Acrylates /C₁₀₋₃₀ Alkyl/Acrylate Crosspolymer.

6. Zubereitungen nach einem der vorstehenden Ansprüche, **dadurch gekennzeichnet, daß** sie 0,001 Gew.-% bis 10 Gew.-%, bevorzugt 0,01 Gew.-% bis 1,0 Gew.-%, insbesondere 0,05 - 0,5 Gew.-%, bezogen auf das Gesamtgewicht der Zubereitungen, an 3-(4-Hydroxy-3-methoxyphenyl)-1-(4-hydroxyphenyl)-propan-1-on enthalten.

7. Zubereitungen nach einem der vorstehenden Ansprüche, **dadurch gekennzeichnet, daß** sie 0,001 Gew.-% bis 30 Gew.-%, bevorzugt 0,05 Gew.-% bis 10 Gew.-%, insbesondere 0,1 - 3,0 Gew.-%, bezogen auf das Gesamtgewicht der Zubereitungen, an einem oder mehreren Emulgatoren.

8. Zubereitungen nach einem der vorstehenden Ansprüche, **dadurch gekennzeichnet, daß** das Verhältnis von 3-(4-Hydroxy-3-methoxyphenyl)-1-(4-hydroxyphenyl)-propan-1-on zu Emulgator (bzw. der Gesamtmenge an Emulgatoren) aus dem Bereich 10:1 bis 1:1000, insbesondere 1:1 bis 1:200 gewählt wird.
